# EUROPEAN PATENT SPECIFICATION

(11) **EP 4 593 790 B1**
(45) Date of publication and mention of the grant of the patent: **18.03.2026**
(21) Application number: 23731610.4
(22) Date of filing: 07.06.2023
(51) Int. Cl.: A61K 8/73, C11D 3/22, C11D 3/00, C11D 1/94, A61K 8/46, A61K 8/44, A61Q 19/10, C11D 1/28, C11D 1/90

(54) **MILD WASH COMPOSITION WITH BIODEGRADABLE THICKENER AND ENHANCED MICROBIOTA IMPACT**
MILDE WASCHZUSAMMENSETZUNG MIT BIOLOGISCH ABBAUBAREM VERDICKUNGSMITTEL UND VERBESSERTER MIKROBIOTA-WIRKUNG
COMPOSITION DE LAVAGE DOUCE AVEC ÉPAISSISSANT BIODÉGRADABLE ET IMPACT AMÉLIORÉ DU MICROBIOTE

(30) Priority: 27.09.2022 EP 22197980
(43) Date of publication of application: 06.08.2025
(73) Proprietor: Unilever IP Holdings B.V., 6708 WH Wageningen (NL); Unilever Global IP Limited, Wirral, Merseyside CH62 4ZD (GB)
(72) Inventor: VASUDEVAN, Tirucherai ,Varahan, 6708 WH Wageningen (NL); VELEZIS, Nicholas, Arthur, 6708 WH Wageningen (NL); YUAN, Mingjun, 6708 WH Wageningen (NL)
(74) Representative: Askew, Sarah Elizabeth
(86) International application number: PCT/EP2023/065240
(87) International publication number: WO 2024/068056

(56) References cited:
- WO-A1-2021/245159
- US-A1- 2020 170 918

## Description

### Field of the Invention

The present invention is directed to a mild and stable wash composition comprising biodegradable thickeners. More particularly, the invention is directed to a wash composition comprising water, surfactant and biodegradable thickener comprising greater than 50% by weight starch based on total weight of thickener. The wash composition is surprisingly mild yet has a pH of 4.25 or less which is ideal for skin microbiota to preserve skin health. The composition also surprisingly yields excellent lather and viscosity characteristics, rinses easily for a clean and filmless sensory feel, and is free of syneresis, discoloration and malodor after being stored at elevated temperatures, even when formulated substantially free of soaps, sulfates, isethionates, thiols and/or synthetic thickeners like ammonium acryloyldimethyltaurate vinylpyrrolidone copolymer, alkyl acrylate cross polymers or the like.

### Background of the Invention

Wash compositions are typically employed to cleanse surfaces, like skin, and often to reduce shine associated with sebum produced in specialized epithelial cells known as sebocytes. They are also used to minimize harmful bacteria on surfaces, like the hands and face, whereby washing is viewed as the most effective way to prevent the spread of germs and bacteria. In fact, experts believe that periodic washing throughout the day can reduce the number of consumers catching colds by about 50%.

Consumers know it is generally good practice to regularly clean their hands and not touch their face, eyes and mouth in order to minimize the risk of getting sick. Information provided by the Centers for Disease Control and Prevention suggests that the COVID-19 (coronavirus) pandemic is a direct result of a virus that is more efficient and severe than influenza and that spreads rapidly from person to person via respiratory droplets. During such a pandemic, consumers understand and medical professionals emphasize it is in everyone's best interest to consistently wash their hands and face, and inanimate surfaces they come in contact with prior to use.

With consumers washing more often, it is highly desirable to develop wash compositions that, are mild and enjoyed by consumers, create positive conditions for skin microbiota and that are friendlier towards the environment. This invention, therefore, is directed to a mild and stable wash composition comprising water, surfactant and biodegradable thickener comprising greater than 50% by weight starch based on total weight of thickener. The wash composition has a pH of 4.25 or less which is ideal for skin microbiota to preserve skin health (i.e., by reducing *Propionibacterium acne,* a main contributor to the pathogenesis of acne). Additionally, and unexpectedly, the composition yields excellent lather and viscosity characteristics, rinses easily for a clean and filmless sensory feel, and is free of syneresis, discoloration and malodor after being stored at elevated temperatures, even when formulated substantially free of soaps, sulfates, isethionates, thiols and synthetic thickeners like ammonium acryloyldimethyltaurate vinylpyrrolidone copolymer, alkyl acrylate cross polymers or the like.

### Additional Information

Wash compositions with thickeners have been disclosed. In U.S. Patent Application No. 2019/0231665A1, hair cosmetic compositions with thiol-based compounds and thickeners are described.

Additional efforts have been disclosed for making wash compositions. In U.S. Patent Application No. 2019/008738A1, personal care compositions with cationic surfactant, nonionic surfactant and a polyacrylate cross polymer structuring agent are described. The wash compositions can include carrageenan as a thickener.

Other efforts have been disclosed for making wash compositions. In U.S. Patent Application No. 2019/0359735A1, water soluble hybrid polymers suitable for use in a body wash are described. Still other efforts have been disclosed for making wash compositions. In U.S. Patent No. 9,549,885, scalp care compositions that can include synthetic rheology modifiers like acrylamide/ammonium acrylates/C10-C30 alkyl acrylate crosslinked polymer or acrylates/steareth-20 itaconate copolymer are described. US2020170918 A1 discloses a cosmetic wash composition comprising 1-30% surfactant, 0.1-5% guaran (guar gum), 0.1-5% xanthan gum, 0-10% unmodified starch and 0-10% modified starch, whereby the total weight of unmodified and modified starch is from 0.1-10%. The aim of the document is to provide thickened wash compositions using renewable raw materials. The pH of the wash compositions is not defined. WO2021245159 A1 explains on page 16 that thickening agents such as starch, cellulose and derivatives thereof or gums, are suitable for thickening systems at pH 4.8 to 6.5.

None of the additional information describes a composition with biodegradable thickener comprising greater than 50% by weight starch based on total weight of thickener, cellulose and/or gum and a pH of 4.25 or less as claimed herein.

### Summary of the Invention

In a first aspect, the present invention is directed to a wash composition comprising:
a) anionic surfactant;
b) amphoteric surfactant, zwitterionic surfactant or both;
c) 0 to 4% by weight nonionic surfactant; and
d) 1.5 to 9.5% by weight thickener, the thickener comprising greater than 50% by weight starch, and preferably at least 70% by weight starch, and most preferably, at least 75% (or 80 to 92% or 82 to 90% or 83 to 88%) by weight starch based on total weight of thickener, with the proviso that the thickener further comprises gum, cellulose or both and where gum makes up from 0.05 to 0.125%, and preferably, from 0.06 to 0.115%, and most preferably, from 0.075 to 0.110% (or 0.8 to 0.105%) by weight of the composition and cellulose makes up from 0.2 to 2.0%, and preferably, from 0.25 to 1.65%, and preferably, from 0.275 to 1.6% (or from 0.28 to 0.8%) by weight of the composition,
wherein the thickener is substantially free of synthetic thickener and the wash composition has a pH of 4.25 or less, and preferably, from 3.4 to 4.2, and most preferably, from 3.65 to 4.15 (or 3.7 to 4.1 or 3.7 to 4 or 3.75 to 3.9) and:
i) the composition has from 3.0 to 16%, and preferably, from 4 to 14%, and most preferably, 4.5 to 12% by weight total surfactant; and
ii) the total surfactant comprises from 20 to 99.5%, and preferably, 30 to 95%, and most preferably, 50 to 85% (or 55 to 80% or from 60 to 75%) by weight anionic surfactant based on total weight of anionic, zwitterionic and/or amphoteric surfactant in the composition,
the composition is substantially free of soap, paraben, formaldehyde donor, thiol, sulfate and/or isethionate.

In a second aspect, the present invention is directed to a wash composition comprising:
a) anionic surfactant comprising a taurate;
b) zwitterionic surfactant comprising a betaine and optionally an amphoteric surfactant;
c) 0 to 4% by weight nonionic surfactant; and
d) 1.5 to 9.5% by weight thickener

e) wherein the thickener comprises greater than 50% by weight starch, and preferably at least 70% by weight starch, and most preferably, at least 75% (or 80 to 92% or 82 to 90% or 83 to 88%) by weight starch based on total weight of thickener, with the proviso that the thickener further comprises a gum, cellulose or both and where gum makes up from 0.05 to 0.125%, and preferably, from 0.06 to 0.115%, and most preferably, from 0.075 to 0.110% (or 0.8 to 0.105%) by weight of the composition and cellulose makes up from 0.2 to 2.0%, and preferably, from 0.25 to 1.65%, and preferably, from 0.275 to 1.6% (or from 0.28 to 0.8%) by weight of the composition,
the composition has from 3.0 to 16%, and preferably, from 4 to 14%, and most preferably, 4.5 to 12% by weight total surfactant; and
   i) the total surfactant comprises from 20 to 99.5%, and preferably, 30 to 95%, and most preferably, 50 to 85% (or 55 to 80% or from 60 to 75%) by weight anionic surfactant based on total weight of anionic, zwitterionic and/or amphoteric surfactant in the composition; and
   ii) the composition has a pH of 4.25 or less, and preferably, from 3.4 to 4.2, and most preferably, from 3.65 to 4.15 (or 3.7 to 4.1 or 3.7 to 4 or 3.75 to 3.9)
wherein the composition is substantially free of synthetic thickener (like ammonium acryloyldimethyltaurate vinylpyrrolidone copolymer, alkyl acrylate crosspolymers or the like).

In a third aspect, the invention is directed to a method of using the compositions according to the first two aspects of the invention to wash a surface, including hair, skin or an inanimate surface.

In a fourth aspect, the invention is directed to a method of using the compositions according to the first two aspects of the invention as a mild wash to enhance skin health wherein the compositions have a pH from 3.4 to 4.2, or preferably from 3.7 to 4.1.

All other aspects of the present invention will more readily become apparent from the description and examples which follow.

Skin, as used herein, is meant to include skin on the arms (including underarms), face, feet, neck, chest, hands, legs, buttocks and scalp (including hair). Cellulose includes a water insoluble polysaccharide composed of chains of glucose molecules linked via *beta 1-4* glycosidic bonds or linkages. Cellulose also includes polysaccharide that is water soluble when chemically or physically modified and water dispersible when used with cellulose that is water soluble. Starch, as used herein, means a water-soluble polysaccharide made up of glucose molecules that are joined by linkages that include *alpha* 1-4 and/or 1-6 glycosidic bonds or linkages. Gum means a thickening agent which can be, for example, a polysaccharide produced by the fermentation of carbohydrates using a gram-negative bacterium (i.e., *Xanthomonas campestris),* a soluble fiber having a protein content typically ranging from 5 to 6% obtained from the endosperm of guar seeds or the like. Wash composition means a composition suitable for use on a surface, including a hard surface such as a table, countertop, appliance, window, ceramic fixture (like a toilet or sink), automobile and floor. Surface also includes skin, and preferably, is skin. Substantially free means less than 0.5%, and preferably, less than 0.2%, and more preferably less than 0.1%, and most preferably, less than 0.05% by weight of the composition. In an embodiment of the invention, substantially free of includes 0.0% (none) by weight of the composition. As to isethionate, substantially free means substantially free of isethionate as a surfactant, not salt like sodium isethionate.

The wash composition of the present invention, therefore, is suitable to be a home care composition, shampoo, make-up remover, facial wash or personal care and liquid body or hand wash. Preferably, the wash composition of the present invention is a body wash or hand wash that is ready for topical application and to be wiped or washed off, and preferably, washed off with water. The wash composition may, optionally, comprise medicinal or therapeutic agents, but preferably, is a wash which is a cleaning and/or cosmetic wash that is a non-therapeutic wash to wash off, for example, dirt, oils, and/or bacteria that can cause staining and/or malodour. As hereinafter described, the wash composition of the present invention may optionally comprise skin benefit ingredients added thereto such as vitamins and/or derivatives thereof, resorcinols, retinoic acid precursors, colorants, moisturizers, sunscreens, mixtures thereof or the like. The skin benefit ingredients (or agents) may be water or oil soluble. If used, oil soluble skin benefit agents typically make up to 1.5% by weight of the wash composition whereby water-soluble skin benefit agents, when used, can make up to 10% by weight of the wash composition. The wash composition typically has a pH of 4.25 or less, and preferably, from 3.4 to 4.2, and most preferably, 3.65 to 4.15 (or 3.7 to 4.1 or 3.7 to 4 or 3.75 to 3.9). Viscosity, unless noted otherwise, is taken with a Discovery HR-2 Rheometer using sand blasted plates having a 1000 micron gap and a first shear rate S_{A} of 4 s⁻¹ for a first viscosity V_{A} and a second shear rate S_{B} of 10 s⁻¹ for a second viscosity V_{B}, both at 25°C and 20 second intervals. Viscosity is reported in millipascal seconds (1 millipascal seconds = 1 centipoise (cps)). Stable, as used herein, means no phase separation, discoloration and malodour generation from the wash composition after being stored for at least two (2) weeks at 50°C, and preferably, at least one (1) month at 45°C, and more preferably, from 1 to 4 months at 45 °C. Rinses easily for a clean and filmless sensory feel means washes off easily without leaving a sticky or tacky residue feeling as determined by trained panelists. Biodegradable, as used herein, means the breakdown of organic matter like starch, gum and cellulose by microorganisms, such as bacteria and fungi. Enhance skin health means providing a composition that is stable, mild and lathers well while simultaneously being within an acidic range (i.e., 4.2 or under) to keep skin microbiome in balance for maintaining healthy skin. Particularly, enhancing skin health means formulated at a pH below the pH of skin (about 5.75) to impact negative bacterium, like *Propionibacterium acne* ,while simultaneously being mild and thus not irritating the skin of a consumer using the wash composition.

The term comprising is meant to encompass the terms consisting essentially of and consisting of. For the avoidance of doubt, and for illustration, the composition of this invention comprising surfactant and biodegradable thickener is meant to include a composition consisting essentially of the same and a composition consisting of the same. Low surfactant level means no more than 16% by weight, and preferably, no more than 14% by weight, and most preferably, no more than 12% (or 8.5%) by weight based on total weight of the composition.

Except in the operating comparative examples, or where otherwise explicitly indicated, all numbers in this description indicating amounts or ratios of materials or conditions and/or physical properties of materials and/or use are to be understood as modified by the word "about". The disclosure, as prepared herein, is meant to support all options and embodiments found in the claims as being multiply dependent upon each other irrespective of the fact that claims may be presented without multiple dependency. All ranges defined herein are meant to include all ranges subsumed therein unless otherwise stated.

### Detailed Description of the Invention

As to the biodegradable thickener suitable for use, the same comprises greater than 50% by weight starch based on total weight of thickener, and cellulose and/or gum where the thickener is not required to be synthetically manufactured and can completely decompose or disintegrate in the environment. In an embodiment of the invention, the thickener has at least 70% by weight starch, and most preferably, at least 75% (or 80 to 92% or 82 to 90%) by weight starch based on total weight of thickener, with the proviso that the thickener does further comprises gum, cellulose or both.

In another embodiment, when both gum and cellulose are used, the weight ratio of gum to cellulose is 1:10 to 10:1, or preferably, from 1:6 to 6:1, or most preferably, from 1:3 to 3:1 (or 1:2 to 2:1 or 1:1.5 to 1.5:1 or 1:1.2 to 1.2:1). In still another embodiment, the weight ratio of gum to cellulose (when both are used with starch) is 1:1. In still another embodiment, the thickener used in the wash composition is at least 95%, and preferably 96 to 100% by weight starch, and gum and/or cellulose based on total weight of thickener used. Most preferably, the thickener used in the wash composition is 100% by weight (all) starch, and gum and/or cellulose.

Typically, the wash composition of the present invention will comprise from 1.5 to 9.5% by weight of the thickener, and preferably, from 3 to 8.5%, and most preferably, from 3.5 to 7.0% (or 4 to 6.2%) by weight of the thickener. In another embodiment of the invention, the thickener makes up from 3.75 to 5.75 or from 3.85 to 5.65% by weight of the wash composition. In an embodiment of the invention and for the avoidance off doubt, 100% by weight of thickener used in the wash composition can be (preferably is) biodegradable thickener having starch and gum and/or cellulose and where the thickener used is not starch free as described herein. In a further embodiment,100% by weight of the biodegradable thickener is starch and cellulose or starch and gum or starch, cellulose and gum whereby the same makes up from 4.75 to 6.25%, and preferably, from 4.85 to 6.15%, and most preferably, from 5 to 5.75% by weight of the wash composition. Again, as to the amount of gum and/or cellulose used, gum typically makes up from 0.05 to 0.125%, and preferably, from 0.06 to 0.115%, and most preferably, from 0.075 to 0.110% (or 0.8 to 0.105%) by weight of the wash composition and cellulose typically makes up from 0.2 to 2.0%, and preferably, from 0.25 to 1.65%, and preferably, from 0.275 to 1.6% (or from 0.28 to 0.8%) by weight of the composition. The amount of starch in the wash compositions should be greater than 0.70%, and preferably, greater than 1%, and most preferably, greater than 1.5%, and most preferably, from 3.5 to 5.75% (or from 3.75 to 5.5% or from 3.8 to 5.2%) by weight of the wash composition.

Salt or electrolyte, like Na, Mg, Ca and/or ammonium chloride make(s) up from 0 to 2.5%, and preferably, from 0.1 to 1.35%, and most preferably, from 0.2 to 1% by weight of the wash composition. In another embodiment, the wash composition comprises less than 0.5% or less than 0.25% by weight or no (0.0% by weight) salt or electrolyte.

As to the biodegradable thickeners suitable for use, the starches include those that may be chemically or physically modified by one or more art recognized techniques like oxidations, crosslinking reactions, gelatinizations, esterification, etherification, amidation and art recognized treatments that include temperature variations. Distarch phosphates or compounds rich in distarch phosphate are starches often included for use. Starch hydrosylates are also suitable for use.

Such starches may originate or be derived from any plant source like corn, pea, potato, oat, rice, tapioca, sorghum, barley or wheat.

Starches suitable for use include starch and distarch phosphates like acetylated distarch phosphate, acetylated distarch adipate, starch sodium octenyl succinate, hydroxypropyl starch phosphate, hydroxypropyl distarch phosphate mixtures thereof or the like. Illustrative examples of starches suitable for use either alone or in mixtures include: StarDesign^{™} Care, StarDesign^{™} Care AV, StarDesign^{™} 05340, C☆EmTex^{®} 06328, PolarTex^{®}, C☆PolarTex^{®}, C☆HiForm^{™}, C☆HiForm A^{™}, HiForm Starch^{™}, StarDesign^{™}, Pure-Gel^{®} B980, Pure-Gel^{®} B990, Pure-Gel^{®} B992, Pure-Gel^{®} B994, FARMAL^{®} AF 1100, FARMAL^{®} CS 3757, FARMAL MS 6822, FARMAL^{™} GMS 2143, FARMAL^{®} MS 6892, FARMAL^{®} MS 6135, FARMAL^{®} GMS 2141, FARMAL^{®} CS 21A, FARMAL^{®} CS 21R, FARMAL^{®} MS 6135, FARMAL^{®} GS 1653, FARMAL^{®} CS 3757, FARMAL^{®} GMS 2141, FARMAL^{®} CS 3001, FARMAL CS 3410, FARMAL^{®} CS 3400, FARMAL^{®} CS 3650, FARMAL^{®} WS 4400, FARMAL^{®} GMS 2141, Nativacare^{™} 8600, Nativacare^{™} 5600, Nativacare^{™} 9360, Nativacare^{™} 9230, Nativacare^{™} 9330; AGENAFLO 9050, AGENAFLO OS 9051, AGENAFLO TS, AGENAJEL 20.313, AGENAJEL 20.306, AGENAJEL 20.350, AGENAMALT 20.222, AGENANOVA 30.326, DEXTRIN 20.901, AGENABON 20.219, STRUCTURE^{®} XL, STRUCTURE^{®} 2143, STRUCTURE^{®} 6892, STRUCTURE^{®} STYLE or the like.

Hydroxypropyl distarch phosphate, sold as E1440 hydroxypropyl starch phosophate by Sinofi, is also suitable for use.

The preferred hydroxypropyl starch and distarch phosphates suitable for use include those made available from Grain Processing Corporation, Pure-Gel^{®} (e.g., B980, B990 food starch modified grade), StarDesign^{™} (e.g., C☆EmTex^{®}), from Cargill Corporation, Agenaflo (9050, 9051) by Agrana, Farmal^{®} (e.g., CS 3757) from Ingredion and (e.g., Zea Mays ST 005) by Roquette.

Other preferred starch phosphates, and in particular hydroxypropyl starch phosphates, or compounds rich in starch phosphate suitable for use are sold by Avebe under the Prejel^{™} name. These include VA-70-T AGGL (gelatinized hydroxypropyl cassava distarch phosphate), TK1 (gelatinized cassava distarch phosphate) and 200 (gelatinized acetyl cassava distarch phosphate).

As to the celluloses suitable for use as biodegradable thickeners, these include microcrystalline components generally classified as a carboxymethylcellulose, hydroxymethylcellulose, hydroxypropylcellulose, hydroxypropylmethyl cellulose, hydroxyethylcellulose or mixtures thereof.

Illustrative examples of the microcrystalline celluloses that may be used include Vivapur^{®} COS5, Vivapur^{®} COS6, Vivapur^{®} COS8, Vivapur^{®} CS 032 XV, Vivapur^{®} CS 152 HV, Vivapur^{®} CS 302 SV, Vivapur^{®} 12, Vivapur^{®} 14, Vivapur^{®} 101, Vivapur^{®} 102, Vivapur^{®} 301, Vivapur^{®} 200, Emcocel^{®} 50M, Emcocel^{®} 90M, Emcocel^{®} 90HD, Emcocel^{®} 200 LP made available from JRS Pharma GmbH & Co.

Others suitable for use include Avicel^{®} GP1412; Avicel^{®} 101, Avicel^{®} 102, Avicel^{®} 103, Avicel^{®} 112, Avicel^{®} 113, Avicel^{®} 301, Avicel^{®} 200, Avicel^{®} SMCC 50, Avicel^{®} SMCC 90, Avicel^{®} SMCC HD90, Avicel^{®} CL, Avicel^{®} DG, Avicel^{®} CE, Avicel^{®} HFE, Lattice^{®} NT-20, Lattice^{®} NTC-61, Lattice^{®} NT-50, Lattice^{®} NT-80, Lattice^{®} NT-100, Lattice^{®} NT-200, GRINDSTED^{®} Cellulose gum BAK, GRINDSTED^{®} Cellulose gum BEV, GRINDSTED^{®} Cellulose gum MAS, GRINDSTED^{®} Cellulose gum AMD, GRINDSTED^{®} Cellulose gum NMD, GRINDSTED^{®} Cellulose gum FZD, Methocel^{™} 240, Methocel^{™} E5, Methocel^{™} F4M, Methocel^{™} K4M, Methocel^{™} K15M, Methocel^{™} K15LV, Methocel^{™} F50, Methocel^{™} K100M, Methocel^{™} K 200M, Methocel^{™} 40-0100, Methocel^{™} 40-0202 and Methocel^{™} F50 and available from Dupont.

Comprecel^{®} 101, Comprecel^{®} 102, Comprecel^{®} 301, Comprecel^{®} 302 available from Ming Thai Chemical Co.; Microcel^{®} 101, Microcel^{®} 102, Microcel^{®} 200 available from Blanver, Farmoquimica; Farmal^{®} CMC 2700 F available from Ingredion; Arbalon^{®} R49, Arbalon^{®} R50 available from Lubrizol; Natrava^{®} Citrus Fibers, Arbalon^{®} Cellulose Liquid available from CP Kelco are also suitable for use. Even other celluloses that can be used include Exilva^{®} FM 02-V available from Borregaard; CelluForce NCC^{®} available from CelluForce; CELOVA^{®}; FiberDesign^{™} Sensation available from Cargill, Natrava^{®} Citrus Fiber available from by CPKelco; CELLOSIZE^{™} QP-10000H EUR, CELLOSIZE^{™} QP-100MH, CELLOSIZE^{™} QP-300, CELLOSIZE^{™} QP-4400H, EUR CELLOSIZE^{™} QP-15000H, CELLOSIZE^{™} QP-30000H, CELLOSIZE^{™} QP-52000H, CELLOSIZE^{™} EP-09 made available by Dow Chemical; Methyl Cellulose PMK-40YS and PMK-60YS available by First Continental International Inc; Carboxymethyl Cellulose (CMC), Poly Anionic Cellulose (PAC), Hydroxyethyl Cellulose (HEC), Hydroxypropyl Methylcellulose (HPMC), and Methyl Hydroxyethyl Cellulose (MHEC/HEMC) made available by Kingsun Chemicals Inc.; Hydroxypropyl MethylCellulose (HPMC), Methyl Cellulose (MC), Sodium CarboxymethylCellulose (CMC), High Substitute Hydroxypropyl Cellulose (H-HPC), Low Substitute Hydroxypropyl Cellulose (L-HPC), Ethyl Cellulose(EC), Hydroxyethyl Cellulose (HEC), Microcrystalline Cellulose (MCC), Polyanionic Cellulose (PAC), Hydroxypropyl Methyl Cellulose Phthalate (HPMC-P)/(Hypromellose phthalate), Hydroxypropyl Methyl Cellulose Acetate Succinate (HPMCAS), Croscarmellose Sodium, Powdered Cellulose made commercially available from SIDLEY CHEMICAL CO., LTD.

The preferred microcrystalline celluloses used in the present invention are those made commercially available from Dupont and JRS Pharma GmbH & Co.

Illustrative guar gums suitable for use in the invention can comprise both mannose and galactose units at a unit ratio of about 2.5:1 to 1.75:1, and preferably, from 2.25:1 to 1.85:1, and most preferably, from 2.1:1 to 1.95:1. The galactose group will be distributed along the mannose chain, and such gums may be nonionic, cationic or anionic.

As to the illustrative nonionic guar gums, the same may be unmodified nonionic guar gums. Often, preferred unmodified and nonionic gums are sold under the names Vidogum GH 175 and 200, Vidogum GHK 175, Vidogum G and Vidocrem, made commercially available by Unipektin. Such guars commercially available from DuPont Danisco, under the name Meypro^{™} LBG Fleur M-175, by Cargill under the Viscogum^{™} name, from Solvay under the name Jaguar^{®} such as Jaguar^{®} S, from Ingredion under the name TICorganic^{®} such as TICorganic^{®} Guar Gum 3500 Powder, and by Polygal AG under the name Polycos such as Polycos N-75 are also suitable for use. Nonionic guar gums made available from suppliers like Ashland and under the name Supercol^{™} US are also within the scope for use in the present invention.

Nonionic guar gums that are modified may be used alone or in combinations with other gums, if desired. Such modified and nonionic guar gums typically have C₁-C₈ hydroxyalkyl groups, preferably hydroxymethyl, hydroxyethyl and/or propyl groups.

Nonionic guar gums often selected for use and modified with hydroxyalkyl groups are sold, for example, under names Jaguar HP60, Jaguar HP 105 and Jaguar HP 120 by Solvay. Other options include those under the name N-Hance^{™} such as N-Hance^{™} HP40 and N-Hance^{™} HP40S made commercially available by Ashland, under the name Polycos such as Polycos A-80, A-80 HV BF, A-240 BF, A-240 HV BF, A-400 HV BF by Polygal AG, and under the names CESMETIC and ESAFLOR such as CESMETIC 4W, ESAFLOR HM 22, ESAFLOR HDR made commercially available by Rita Corp.

Galactomannan gums suitable for use typically have a cationic charge density of less than or equal to 1.65 meq./g, more often between 0.1 and 1.2 meq./g as measured via art recognized (e.g., ASTM) techniques.

Cationic galactomannan gums that may be used in the wash compositions of the invention are, for example, gums comprising tri (C₁₋₄) alkylammonium cationic groups. Usually, 3% to 25% of the total percent of hydroxy functional groups of these gums have trialkylammonium cationic groups. In an embodiment of the invention, a cationic galactomannan gum used is one comprising hydroxypropyltrimethylammonium groups such as guar gum modified with 2,3-epoxypropyltrimethylammonium chloride.

Specific galactomannan gums suitable for optional use are described in U.S. Pat. Nos. 3,589,578 and 4,031,307. Such gums (i.e., guar hydroxypropyltrimonium chlorides) are made commercially available under the name name Jaguar^{®} such as Jaguar EXCEL, Jaguar C13 S, Jaguar C 15, Jaguar C 17 and Jaguar C162 by Solvay, under the name Amilan^{™} by Evonik Degussa, and under the name N-Hance^{™} such as N-Hance^{™} BF13, N-Hance^{™} CG13, N-Hance^{™} CG17, N-Hance^{™} CCG45, N-Hance^{™} C261N, N-Hance^{™} HPCG 1000, N-Hance^{™} HPCG 1000, N-Hance^{™} 3000, N-Hance^{™} 3197, N-Hance^{™} 3215 by Ashland, under the name Polycos such as Polycos CA-45, Polycos CA-750, Polycos CA-100 RS, Polycos CA-1800, Polycos CA-3000, Polycos CA-3002, by Polygal AG, and under the names CESMETIC and ESAFLOR such as CESMETIC BF7, ESAFLOR HC 56, ESAFLOR EC4 USA, ESAFLOR EC3, ESAFLOR EC 5, ESAFLOR BF7 by Rita Corp.

Anionic guar gums suitable for use comprise groups typically derived from carboxylic, sulfonic, sulfenic, phosphoric, phosphonic or pyruvic acid as well as salts thereof. Most preferably, the anionic group is a carboxylic acid group. Counter ions typically include sodium, calcium, or potassium.

The anionic guar gums, when used, are often those classified as carboxymethyl guar derivatives (i.e., carboxymethyl guar or carboxymethyl hydroxypropyl guar).

Another gum suitable for use in the wash composition of this invention is locust bean or carob bean gum, recovered, for example, from the seed kernels of the carob tree (Ceratonia siliqua).

Locust bean gum optional for use is sold, for example, by Cargill under the name Viscogum^{™}; Vidogum L by Unipektin and under the name Grinsted^{®} LBG by DuPont Danisco. Modified locust bean gums that may be used in this invention include, for example, the cationic locust beans sold under the names Catinal CLB, Catinal CTR-100 and Catinal CTR-200 (locust bean hydroxypropyltrimonium chloride) by Toho Chemical.

Tara gums suitable for use in the wash composition of the invention include, for example, those made commercially available under the name Vidogum SP by Unipektin.

In an embodiment of the invention, the gums used will include hydroxypropyltrimethyl ammonium chloride guar, locust bean gum, konjac mannan gum, gum tragacanth, sodium or propylene glycol alginate, kappa-, iota-, or lambda-carrageenan, guar, hydroxylpropyl guar gum, karaya gum, gum arabic (acacia), gellan, xanthan, succinoglycan or its acidic or enzymatic hydrolysates, agar, pectin, mixtures thereof or the like.

In an especially preferred embodiment, the gum selected for use is Jaguar^{®} S from Solvay, TICorganic^{®} Guar Gum 3500 Powder from Ingredion, Viscogum^{™} Guar Gum made commercially available from Cargill and/or Gellan Gum (anionic polysaccharide produced by the bacterium *Shingomonas elodea)* made commercially available from CP Kelco.

The biodegradable thickeners preferred typically have a bulk density from 0.18 to 0.90, and preferably, from 0.2 to 0.8, and most preferably, from 0.22 to 0.70 g/ml (or 1000kg/m³) where bulk density is the ratio of the mass of an untapped powder sample and its volume including the contribution of the interparticulate void volume (i.e., dependent on both the density of powder particles and the spatial arrangement of particles in a powder bed). Such thickeners also have a cloud point from 60 to 70°C and a weight average molecular weight from 75,000 to 1.5 million Daltons, and preferably, from 80,000 to 1.25 million Daltons, and most preferably, from 100,000 to 1.0 million Daltons.

Regarding the surfactants that may be used, the same are limited only to the extent that they are suitable for use in compositions that are topically applied to a surface, preferably skin.

Anionic surfactants suitable for use in the wash composition of the present invention include aliphatic sulfonates, such as a primary alkane (e.g., C₈-C₂₂) sulfonate, primary alkane (e.g., Cs-C₂₂) disulfonate, C₈-C₂₂ alkene sulfonate, C₈-C₂₂ hydroxyalkane sulfonate or alkyl glyceryl ether sulfonate (AGS); or aromatic sulfonates such as alkyl benzene sulfonate. The anionic may also be an alkyl sulfate (e.g., C₁₂-C₁₈ alkyl sulfate) or alkyl ether sulfate (including alkyl glyceryl ether sulfates). Among the alkyl ether sulfates are those having the formula:

RO(CH₂CH₂O)ₙSO₃M

wherein R is an alkyl or alkenyl having 8 to 18 carbons, preferably 12 to 18 carbons, n has an average value of at least 1.0, preferably less than 5, and most preferably 1 to 4, and M is a solubilizing cation such as sodium, potassium, ammonium or substituted ammonium.

The anionic may also include alkyl sulfosuccinates (including mono- and dialkyl, e.g., C₈-C₂₂ sulfosuccinates); acyl taurates (often methyl taurates), acyl sarcosinates, sulfoacetates, C₈-C₂₂ alkyl phosphates and phosphonates, alkyl phosphate esters and alkoxyl alkyl phosphate esters, acyl lactates, C₈-C₂₂ monoalkyl succinates and maleates, alkyl glucosides and acyl isethionates, and the like.

Sulfosuccinates may be monoalkyl sulfosuccinates having the formula:

R¹O₂CCH₂CH(SO₃M)CO₂M;

and amide-MEA sulfosuccinates of the formula:
R¹CONHCH₂CH₂0₂CCH₂CH(SO₃M)CO₂M wherein R¹ ranges from C₈-C₂₂ alkyl.

Sarcosinates are generally indicated by the formula:
R²CON(CH₃)CH₂CO₂M, wherein R² ranges from C₈-C₂₀ alkyl.

Taurates are generally identified by formula:

R³CONR⁴CH₂CH₂SO₃M

wherein R³ is a C₈-C₂₀ alkyl, R⁴ is a C₁-C₄ alkyl.

M is a solubilizing cation as previously described. The isethionates that may be used include C₈-C₁₈ acyl isethionates (including those which have a substituted head group such as a C₁₋₄ alkyl substitution, preferably methyl substitution). These esters are prepared by a reaction between alkali metal isethionate with mixed aliphatic fatty acids having from 6 to 18 carbon atoms and an iodine value of less than 20. Often at least 75% of the mixed fatty acids have from 12 to 18 carbon atoms and up to 25% have from 6 to 10 carbon atoms.

The acyl isethionate suitable for optional use may be an alkoxylated isethionate such as is described in Ilardi et al., U.S. Pat. No. 5,393,466, entitled "Fatty Acid Esters of Polyalkoxylated isethonic acid; issued Feb. 28, 1995. This compound has the general formula:

R⁵C-O(O)-C(X)H-C(Y)H-(OCH₂-CH₂)ₘ-SO₃M

wherein R⁵ is an alkyl group having 8 to 18 carbons, m is an integer from 1 to 4, X and Y are each independently hydrogen or an alkyl group having 1 to 4 carbons and M is a solubilizing cation as previously described.

In an embodiment of the invention, an anionic surfactant suitable for optional use is sodium lauroyl isethionate, sodium cocoyl isethionate, sodium methyl lauroyl taurate, sodium methyl cocoyl taurate or a mixture thereof. Such anionic surfactants are commercially available from suppliers like Galaxy Surfactants, Clariant, Sino Lion and Innospec. Sodium methyl lauroyl taurate, is often the preferred anionic used.

The wash compositions can optionally contain glutamates and glycinates but as is the case with isethionates, they are substantially free (i.e., under 0.5%) of the same in surfactant form. If optionally used, the glycinates suitable include sodium lauroyl glycinate, sodium cocoyl glycinate, sodium lauroyl glutamate, sodium cocoyl glutamate, sodium myristol glutamate, isopropyl lauroyl glutamate (i.e., C₆-C₁₈ glutamates and glycinates) or mixtures thereof.

Amphoteric surfactants suitable for use in the invention (which depending on pH can be zwitterionic) include sodium acyl amphoacetates, sodium acyl amphopropionates, disodium acyl amphodiacetates and disodium acyl amphodipropionates where the acyl (i.e., alkanoyl group) can comprise a C₇-C₁₈ alkyl portion. Illustrative examples of the amphoteric surfactants suitable for use include sodium lauroamphoacetate, sodium cocoamphoacetate, sodium lauroamphoacetate, sodium cocoamphoacetate or mixtures thereof.

As to the zwitterionic surfactants that may be employed in the wash compositions of the present invention, such surfactants include at least one acid group. Such an acid group may be a carboxylic or a sulphonic acid group. They often include quaternary nitrogen, and therefore, can be quaternary amino acids. They should generally include an alkyl or alkenyl group of 7 to 18 carbon atoms generally comply with an overall structural formula:
R⁶-[-C(O)-NH(CH₂)_{q}-]ᵣ₋N⁺-(R⁷-)(R⁸)A-B where R⁶ is alkyl or alkenyl of 7 to 18 carbon atoms; R⁷ and R⁸ are each independently alkyl, hydroxyalkyl or carboxyalkyl of 1 to 3 carbon atoms; q is 2 to 4; r is 0 to 1; A is alkylene of 1 to 3 carbon atoms optionally substituted with hydroxyl, and B is --CO₂-- or --SO₃--.

Suitable zwitterionic surfactants that may be used in the present invention and within the above general formula include simple betaines of formula:

R⁶-N⁺-(R⁷)(R⁸)CH₂CO₂⁻

and amido betaines of formula:
R⁶-CONH(CH₂)ₜ-N⁺-(R⁷)(R⁸)CH₂CO₂⁻ where t is 2 or 3.

In both formulae R⁶, R⁷ and R⁸ are as defined previously. R⁶ may, in particular, be a mixture of C₁₂ and C₁₄ alkyl groups derived from coconut oil so that at least half, preferably at least three quarters of the groups R⁶ have 10 to 14 carbon atoms. R⁷ and R⁸ are preferably methyl.

A further possibility is that the zwitterionic surfactant is a sulphobetaine of formula:

R⁶--N⁺--(R⁷)(R⁸)(CH₂)₃SO₃⁻

or

R⁶-CONH(CH₂)ᵤ -N⁺-(R⁷)(R⁸)(CH₂)₃SO₃⁻

where u is 2 or 3, or variants of these in which --(CH₂)₃SO₃⁻ is replaced by-CH₂C(OH)(H)CH₂SO₃⁻.

In these formulae, R⁶, R⁷ and R⁸ are as previously defined.

Illustrative examples of zwitterionic surfactants suitable for use include betaines like cocodimethyl carboxymethyl betaine, cocamidopropyl betaine and laurylamidopropyl betaine. An additional zwitterionic surfactant suitable for use includes cocamidopropyl hydroxy sultaine. Such surfactants are made commercially available from suppliers like Stepan Company, and it is within the scope of the invention to employ mixtures of the aforementioned surfactants. In a preferred embodiment, the zwitterionic surfactant used in the wash composition of this invention is cocamidopropyl betaine.

Nonionic surfactants may be used in the wash composition of the present invention. When used, nonionic surfactants are typically used at levels as low as 0.01, 0.5, 1, 1.5, 2, 3 or 4% or by weight of the wash composition. The nonionics which may be used include, in particular, the reaction products of compounds having a hydrophobic group and a reactive hydrogen atom, for example aliphatic alcohols, acids, amides or alkylphenols with alkylene oxides, especially ethylene oxide either alone or with propylene oxide. Specific nonionic surfactant compounds are alkyl (C₆-C₂₂) phenols ethylene oxide condensates, the condensation products of aliphatic (Cs-C₁₈) primary or secondary linear or branched alcohols with ethylene oxide, and products made by condensation of ethylene oxide with the reaction products of propylene oxide and ethylenediamine. Other nonionic surfactants include long chain tertiary amine oxides, long chain tertiary phosphine oxides, dialkyl sulphoxides, and the like. Cocamide monoethanolamine (MEA) is an often preferred nonionic surfactant suitable for use at levels from 0.2 to 0.65% by weight of the wash composition.

In an embodiment of the invention, nonionic surfactants optionally used can include fatty acid/alcohol ethoxylates having the following structures a) HOCH₂(CH₂)ₛ(CH₂CH₂O)ᵥ H or b) HOOC(CH₂)_{c}(CH₂CH₂O)_{d} H; where s and v are each independently an integer up to18; and c and d are each independently an integer from 1 or greater. In an embodiment of the invention, s and v are each independently 6 to 18; c and d are each independently 1 to 30. Other options for nonionic surfactants include those having the formula HOOC(CH₂)ᵢ-CH=CH-(CH₂)ₖ(CH₂CH₂O)_{z} H, where i, k are each independently 5 to 15; and z is 5 to 50. In another embodiment of the invention, i and k are each independently 6 to 12; and z is 15 to 35.

The nonionic may also include a sugar amide, such as a polysaccharide amide. Specifically, the surfactant may be one of the lactobionamides described in U.S. Pat. No. 5,389,279 to Au et al., entitled "Compositions Comprising Nonionic Glycolipid Surfactants issued Feb. 14, 1995; or it may be one of the sugar amides described in U.S. Pat. No. 5,009,814 to Kelkenberg, titled "Use of N-Poly Hydroxyalkyl Fatty Acid Amides as Thickening Agents for Liquid Aqueous Surfactant Systems" issued Apr. 23, 1991.

In an embodiment of the invention, cationic surfactants may optionally be used in the wash composition of the present invention.

One class of optional cationic surfactants includes heterocyclic ammonium salts such as cetyl or stearyl pyridinium chloride, alkyl amidoethyl pyrrylinodium methyl sulfate, and lapyrium chloride. Tetra alkyl ammonium salts are another useful class of cationic surfactants suitable for optional use. Examples include cetyl or stearyl trimethyl ammonium chloride or bromide; hydrogenated palm or tallow trimethylammonium halides; behenyl trimethyl ammonium halides or methyl sulfates; decyl isononyl dimethyl ammonium halides; ditallow (or distearyl) dimethyl ammonium halides, and behenyl dimethyl ammonium chloride.

Still other types of cationic surfactants that may be used are the various ethoxylated quaternary amines and ester quats. Examples include PEG-5 stearyl ammonium lactate (e.g., Genamin KSL manufactured by Clariant), PEG-2 coco ammonium chloride, PEG-15 hydrogenated tallow ammonium chloride, PEG 15 stearyl ammonium chloride, dipalmitoyl ethyl methyl ammonium chloride, dipalmitoyl hydroxyethyl methyl sulfate, and strearyl amidopropyl dimethylamine lactate.

Even other useful cationic surfactants suitable for optional use include quaternized hydrolysates of silk, wheat, and keratin proteins, and it is within the scope of the invention to use mixtures of the aforementioned cationic surfactants.

If used, cationic surfactants will make up no more than 1.0% by weight of the wash composition. When present, they typically make up from 0.01 to 0.7%, and more typically, from 0.1 to 0.5% by weight of the wash composition..

In an embodiment of this invention, the wash composition will comprise less than 0.18% polymeric quaternary ammonium compounds (including salts of the same). In another embodiment, the wash composition will comprise less than 0.1% by weight polymeric quaternary ammonium compounds. In yet another embodiment, the wash composition comprises less than 0.01% by weight polymeric quaternary ammonium compounds. In even another embodiment, the wash composition is free of polymeric quaternary ammonium compounds (i.e., 0.0%).

While not desired fatty acid soaps like sodium stearate may be included in the composition but the compositions are substantially free of the same.

Again, the wash composition of the present invention is as herein defined substantially free of soaps, sulfates (e.g., isethionates as surfactants), thiols and/or synthetic thickeners like ammonium acryloyldimethyltaurate vinylpyrrolidone copolymer, alkyl acrylate cross polymers or the like. The wash compositions are also substantially free of glutamates and glycinates as surfactants as well as sulfates like sodium lauryl, laureth and pareth sulfate.

As to the composition surfactant amounts, the same has from 3.0 to 16%, and preferably, from 4 to 14%, and most preferably, 4.5 to 12% (or 4.5 to 9.5% or 4.6 to 8.75% or 4.75 to 7.5%) by weight total surfactant and the total surfactant comprises from 20 to 99.5%, and preferably, 30 to 95%, and most preferably, 50 to 85% (or 55 to 80% or from 60 to 75%) by weight anionic surfactant based on total weight of anionic, zwitterionic and/or amphoteric surfactant in the composition. In even another embodiment, the weight ratio of anionic surfactant (preferably, sodium methyl lauroyl taurate) to zwitterionic surfactant (preferably, cocoamidopropyl betaine) is from 1:1 to 1: 2.2, and preferably, from 1:1.2 to 1:2.1, and most preferably, from 1:1.5:1.35 to 1:1.85. Typically, 8 to 38, and preferably, 9 to 35, and most preferably, 10 to 32 times more starch is used than cellulose, or gum or the combination of cellulose and gum.

Water typically makes up from 62 to 95%, and preferably, from 65 to 90%, and most preferably, from 65 to 80% by weight of the wash composition.

Adjusters suitable to modify/buffer the pH may optionally be included. Such pH adjusters include triethylamine, NaOH, KOH, H₂SO₄, HCl, C₆H₈O₇ (i.e., citric acid) or mixtures thereof. The pH adjusters are added at amounts to yield the desired final pH, typically 0 to 3.5 by weight of the composition. The pH values may be assessed with commercial instrumentation such as a pH meter made commercially available from Thermo Scientific^{®}. Such pH values of the wash composition typically are 4.25 or less, and preferably, from 3.4 to 4.2, and most preferably, from 3.65 to 4.15 (or 3.7 to 4.1 or 3.7 to 4 or 3.75 to 3.9).

Polyols suitable for use in the wash composition are limited only to the extent that they are usable in a wash composition for topical application. Illustrative and non-limiting examples of the polyols suitable for use in the present invention include sorbitol, glycerol, mannitol, xylitol, maltitol or mixtures thereof. In an embodiment of the invention, the polyol used is typically at least 50% by weight glycerol, based on total weight of the polyol used in the wash composition. In another embodiment of the invention, the polyol used is all glycerol (100% by weight). Polyol will typically make up from 0.0 to 25% by weight of the wash composition, and preferably, from 0.5 to 8% by weight of the wash composition, and most preferably, from 0.75 to 3.5% by weight of the wash composition.

While not required, in addition to the mixture of thickener having starch and cellulose of the present invention, it is within the scope of this invention to optionally use traditional gelling or thickening agents like ammonium acryloyldimethyltaurate vinylpyrrolidone copolymer, alkyl acrylate cross polymers or the like. If used, the acrylates include those generally classified as acrylic acid crosslinked with allyl sucrose or allyl pentaerythritol acrylic acid and C₁₀-C₃₀ alkyl acrylate crosslinked with allyl pentaerythritol. Such acrylates are sold under the Carbopol^{®} and Ultrez^{®} names and are made commercially available by Lubrizol. Other acrylates suitable for use include those which are a copolymer formed from an ester of acrylic acid and ethoxylated palm alcohol with about 25 moles of ethylene oxide and one or more monomers of acrylic acid, methacrylic acid or one of their simple esters. These acrylates are sold, for example, under the Synthalen^{®} (i.e., W200, W2000) names, and made commercially available from suppliers like 3V Sigma USA. When used such optional thickeners will make up less than 0.2%, and preferably, less than 0.1%, and most preferably, less than 0.05% by weight of the composition. Again, and even more preferably, no (0.0%) thickener in addition to starch and cellulose as described herein is used.

The viscosity of the wash composition (measured at 4 s⁻¹ for 20 seconds) is typically 40,000 cps or less, and preferably, from 700 to 15,000 cps, and most preferably, from 2000 to 13,500 cps.

Optional skin benefit agents suitable for use in the wash composition are limited only to the extent that they are capable of being topically applied, and suitable to remain stable in the wash composition at the desired pH.

Illustrative examples of the benefit agents suitable to include in the water portion of the wash composition are acids, like amino acids, such as arginine, valine or histidine. Additional water soluble benefit agents suitable for use include vitamin B₂, niacinamide (vitamin B₃), vitamin B5 (pantothenic acid), vitamin B₆, vitamin C, mixtures thereof or the like. Water soluble derivatives of such vitamins may also be employed. For instance, vitamin C derivatives such as ascorbyl tetraisopalmitate, magnesium ascorbyl phosphate and ascorbyl glycoside may be used alone or in combination with each other. Other water-soluble benefit agents suitable for use include 4-ethyl resorcinol, extracts like sage, aloe vera, green tea, grapeseed, thyme, chamomile, yarrow, cucumber, liquorice, rosemary extract or mixtures thereof. Water soluble sunscreens like ensulizole may also be used. Total amount of optional water-soluble benefit agents (including mixtures) when present in the wash composition may range from 0.0 to 10%, preferably from 0.001 to 8%, and most preferably, from 0.01 to 6% (or 0.01 to 2%) by weight, based on total weight of the wash composition.

It is also within the scope of the present invention to optionally include oil soluble benefit agents. Such oil soluble actives or benefit agents can be solubilized in the surfactants used. The only limitation with respect to such oil soluble benefit agents are that the same are suitable to provide a benefit when topically applied.

Illustrative examples of the types of oil soluble benefit agents that may optionally be used in the compositions of this invention include components like stearic acid, vitamins like Vitamin A, D, E and K (and their oil soluble derivatives), sunscreens like ethylhexylmethoxycinnamate, bis-ethyl hexyloxyphenol methoxyphenol triazine, 2-ethylhexyl-2-cyano-3,3-diphenyl-2-propanoic acid, drometrizole trisiloxane, 3,3,5-trimethyl cyclohexyl 2-hydroxybenzoate, 2-ethylhexyl-2-hydroxybenzoate or mixtures thereof.

Other optional oil soluble benefit agents suitable for use include resorcinols like 4-hexyl resorcinol, 4-phenylethyl resorcinol, 4-cyclopentyl resorcinol, 4-cyclohexyl resorcinol 4-isopropyl resorcinol, thiamidol (Isobutylamido thiazolyl resorcinol) or a mixture thereof. Also, 5-substituted resorcinols like 4-cyclohexyl-5-methylbenzene-1,3-diol, 4-isopropyl-5-methylbenzene-1,3-diol, mixtures thereof or the like may be used. The 5-substituted resorcinols, and their synthesis are described in commonly assigned U.S. Published Patent Application No. 2016/0000669A1.

Even other oil soluble actives suitable for use include omega-3 fatty acids, omega-6 fatty acids, climbazole, farnesol, ursolic acid, myristic acid, geranyl geraniol, oleyl betaine, cocoyl hydroxyethyl imidazoline, hexanoyl sphingosine, 12-hydroxystearic acid, petroselinic acid, conjugated linoleic acid, terpineol, thymol mixtures thereof or the like.

In an embodiment of the invention, the optional oil soluble benefit agent used is a retinoic acid precursor. In one embodiment of the invention, the retinoic acid precursor is retinol, retinal, retinyl propionate, retinyl palmitate, retinyl acetate or a mixture thereof. Retinyl propionate, retinyl palmitate and mixtures thereof are typically preferred.

When optional oil soluble active or benefit agent is used in the composition, such active typically makes up from 0.0001 to 1.5%, and preferably, from 0.001 to 0.65%, and most preferably, from 0.05 to 0.35% by weight of the wash composition. In yet another embodiment, oil soluble benefit agent makes up from 0.1 to 0.5% by weight of the total weight of the wash composition.

Other optional additives suitable for use include zinc pyrithione, octopirox and mixtures thereof. When used, they typically are employed from 0.001 to 1.5%, and preferably, from 0.01 to 1% by weight of the wash composition.

Conventional preservatives can desirably be incorporated into the wash composition to protect against the growth of potentially harmful microorganisms. Cosmetic chemists are familiar with appropriate preservatives and routinely choose them to satisfy the preservative challenge test and to provide product stability. Suitable traditional preservatives for use include hydantoin derivatives and propionate salts. Particularly preferred preservatives are iodopropynyl butyl carbamate, phenoxyethanol, hydroxyacetophenone, ethylhexylglycerine, hexylene glycol, methyl paraben, propyl paraben, imidazolidinyl urea, sodium dehydroacetate, dimethyl-dimethyl (DMDM) hydantoin, sodium benzoate, benzyl alcohol and mixtures thereof. Other preservatives suitable for use include sodium dehydroacetate, chlorophenesin, decylene glycol, N-capryloyl glycine, N-undecylenoyl glycine and mixtures of the same. The preservatives should be selected having regard for the use of the composition and possible incompatibilities between the preservatives and other ingredients in the composition. Preservatives are preferably employed in amounts ranging from 0.01% to 2.0% by weight of the total weight of the wash composition. A preservative system that includes from 0.1 to 1.0% of 1,2-octane diol (i.e., conventional diol emollient, or the like) based on total weight of the wash composition is suitable for use. Preservative systems substantially free of parabens and hydantoins are preferred. Sodium benzoate is an often most preferred preservative and employed typically at from 0.35 to to 2.65% or 2.0 to 1.45% by weight of the wash composition.

Fragrances, fixatives, chelators (like EDTA), opacifiers (like titanium dioxide and vegetable-derived liquid dispersions like MACKADET^{®} OPR 2 made available from Solvay), inorganics and exfoliants may optionally be included in the wash composition. Each of these substances may range from about 0.03 to about 5%, preferably between 0.1 and 3%, and most preferably, from 0.1 to 2% by weight of weight of the wash composition. To the extent the exfoliants are used, those selected should be of small enough particle size so that they do not impede the performance of any packaging used to dispense the compositions of this invention. Conventional antimicrobial agents, like benzalkonium and/or benzethonium chloride, salicylic acid, cetrimonium chloride are also suitable for use, typically at amounts from 0.01 to 1%, and preferably, from 0.05 to 0.6%, and most preferably, from 0.1 to 5% by weight of the composition.

Conventional emulsifiers having an HLB of greater than 8 may optionally be used. Illustrative examples include Tween, 40, 60, 80, polysorbate 20 and mixtures thereof. Such emulsifiers, when used for water continuous systems, make up from 0.03 to 1.5% by weight of the wash composition.

Regarding silicones like dimethicone or the like, the wash compositions will typically comprise less than 1.0% by weight silicone, more preferably, less than 0.5% by weight silicone, and most preferably, no silicone. In an embodiment of the invention, less than 0.5%, or less than 0.25%, or less 0.1% or no (0.0%) decamethylcyclopentasiloxane is used in the wash composition.

The wash composition of the present invention is preferably a liquid wash composition suitable to be in an isotropic or lamellar phase.

When lamellar, the composition of the invention utilizes from 0.1 to 20%, and preferably, from 1 to 10%, and most preferably from 2 to 5% by weight of a lamellar structuring agent (based on total weight of liquid wash composition) which works in the compositions to form the lamellar phase.

Examples of fatty acids (or ester derivative, or fatty alcohol, or trihydroxystearin) which may be used as structurants are C₈-C₂₂ fatty acids like caprylic acid, lauric acid, myristic acid, oleic acid, isostearic acid, linoleic acid, linolenic acid, ricinoleic acid, elaidic acid, arachidonic acid, myristoleic acid and palmitoleic acid, mixtures thereof or the like. Ester derivatives include propylene glycol isostearate, propylene glycol oleate, glyceryl isostearate, glyceryl oleate and polyglyceryl diisostearate, or the like. The structurant is preferably a fatty acid. More preferably, the structurant is selected from the group consisting of caprylic acid, lauric acid, myristic acid or a mixture thereof.

When making wash composition of the present invention, the desired ingredients may be mixed with conventional apparatus under moderate shear and atmospheric conditions, with temperature ranging from 30 to 85°C whereby shear continues until a homogeneous product is recovered.

The packaging for the wash composition typically is not limited as long as composition can be dispensed. In an embodiment on the invention, the wash composition is sold in a pouch, bottle, jar, tube or canister. The packaging preferably allows for infinite numbers of refilling to invariably reduce plastic waste in the environment, and most preferably, has at least 50% by weight post-consumer resin. The wash compositions of the present invention may be sold in a concentrated form whereby the consumer can be instructed to add water to generate product ready for use and in an effort to reduce weight when shipping and over reliance on virgin plastic.

The Examples are provided to facilitate an understanding of the invention. They are not intended to limit the scope of the claims.

### Examples

All Samples in the Examples were prepared by mixing the ingredients with moderate shear. Temperature was varied from about 60 to 78°C and pressure while mixing was atmospheric.

The starch used in the Samples was StarDesign^{™} Care provided commercially from Cargill, the cellulose used was Vivapur^{®} COS6 provided commercially from JRS Pharma GmbH & Co and the gum used was xanthan or carrageenan. Hydroxypropyl starch phosphate used was Puregel B990 made available from Grain Processing Corporation.

### Example I

Base wash compositions were made with the ingredients identified below. Starch, and cellulose and/or gum were added to the base compositions as described in the Samples of Example II.

The wash compositions had a pH from 3.5 to 4.2. The ingredients were added based on total weight of the wash composition.

**Table I**

| Ingredient | Sample Wash 1 (Wt.%) | Sample Wash 2 (Wt.%) | Sample Wash 3 (Wt.%) |
|---|---|---|---|
| Sodium methyl lauroyl taurate | 4.2 | 4.2 | 5.0 |
| Cocamidopropyl betaine | 1.8 | 1.8 | 2.1 |
| Cocomonoethanolamide | 0.5 | - | - |
| Glycerin | 5 | 5 | - |
| Lauric acid | 0.25 | 0.25 | 0.25 |
| Stearic acid | 0.31 | 0.31 | 0.25 |
| 12 Hydroxystearic acid | - | - | 0.25 |
| Salt (NaCl)* | 0 - 2.0 | 0 - 2.0 | 0 |
| Conditioning Polymer | - | - | 0.2 |
| Sodium benzoate | | | 2.5 |
| Benzalkonium chloride | | | 0.13 |
| pH modifiers | To target pH | To target pH | 2.85 |
| Preservative & Minors | 0.5 - 2.0 | 0.5 - 2.0 | 1.0 |
| Natural thickeners | ** | ** | ** |
| water | To 100 | To 100 | To 100 |

| | | | |
|---|---|---|---|
| *Salt added to about 90% detergent neutralization **As Identified in Example II | | | |

### Example II

Completed wash compositions, Example I, Table I (Sample 1) with biodegradable thickener (added in weight percent based on total weight of each thickener as described in Table II) were prepared.

**Table II**

| Starch (wt%) | Cellulose (wt%) | Iota Carageenan (wt%) | Xanthan Gum (wt%) | Guar Gum (wt%) | Stability |
|---|---|---|---|---|---|
| 4.5 | 0.75 | - | - | - | Stable |
| 4.5 | 0.5 | - | - | - | Stable |
| 4.5 | - | 0.1 | - | - | Stable |
| 4.5 | - | - | 0.1 | - | Stable |
| 4.5 | - | - | | 0.1 | Stable |
| 4.0 | 0.3 | | | | Stable |
| 4.3 | | 0.15 | | | Unstable |
| 4.0 | | | | | Unstable |
| 0.5 | | 0.45 | | | Unstable |
| - | 0.15 | 0.4 | | | Unstable |
| - | 1.0 | | 0.8 | | Unstable |
| - | 1.5 | | 0.8 | | Unstable |

Such Sample I from Example I was made with the thickening agents described in Example II. Compositions made according to the present invention unexpectedly were stable in that after being stored for at least two (2) weeks at 50 °C, and 1 to 4 months at 45 °C no malodor, syneresis, or discoloration was observed. The wash compositions made according to the invention were assessed by trained panellists and such composition rinsed off easily for a clean and filmless sensory feel and did not leave a sticky or tacky residue feeling. It was especially surprising that the panelists concluded the compositions (in the acidic pH range) were mild and did not irritate the skin (were not skin sensitizing) even when washing with warm/hot water (about 48°C).

### Example III

Completed wash compositions of Example I, Table I (Sample 2) with biodegradable thickener (added in weight percent based on total weight of each thickener as described in Table II) were prepared.

**Table III**

| Starch diphosphate | Hydroxypropyl starch phosphate | Cellulose | Stability |
|---|---|---|---|
| 4.0 | - | 0.3 | Stable |
| - | 4.0 | 0.15 | Stable |
| - | 4.75 | 0.15 | Stable |

The data in this example unexpectedly demonstrates that compositions according to the invention can be made using starch diphosphate (i.e., crosslinked) or hydroxypropyl starch phosphate (non-crosslinked) in combination with cellulose.

These compositions made according to the present invention unexpectedly were stable in that after being stored for at least two (2) weeks at 50°C, and 1 to 4 months at 45 °C no malodor, syneresis, or discoloration was observed. The wash compositions made according to the invention were assessed by trained panellists and such composition rinsed off easily for a clean and filmless sensory feel and did not leave a sticky or tacky residue feeling. It was especially surprising that the panelists concluded the compositions (in the acidic pH range) were mild and did not irritate the skin (were not skin sensitizing) even when washing with warm/hot water (about 48°C).

### Example IV

Completed wash compositions of Example I, Table I (Sample 3) with biodegradable thickener (added in weight percent based on total weight of each of the thickeners as described in Table III) were prepared.

**Table IV**

| Starch diphosphate | Hydroxypropyl starch phosphate | Cellulose | Stability |
|---|---|---|---|
| 4.0 | - | 0.3 | Stable |
| - | 4.0 | 0.15 | Stable |
| - | 4.75 | 0.15 | Stable |
| - | 3.3 | 0.15 | Stable |
| - | 2.5 | 0.15 | Stable |
| - | 2.0 | 0.15 | Stable |

The data obtained in this Example IV were identical to those described in Example III and they demonstrate that stable wash compositions can be obtained using starch diphosphate (cross linked starches) and hydroxypropyl starch phosphate (non-crosslinked starches) in combination with cellulose when made consistent with the invention.

## Claims

1. A wash composition comprising:
a) anionic surfactant;
b) amphoteric surfactant, zwitterionic surfactant or both;
c) 0 to 4% by weight nonionic surfactant; and
d) 1.5 to 9.5% by weight thickener, the thickener comprising greater than 50% by weight starch, and preferably at least 70% by weight starch, and most preferably, at least 75% (or 80 to 92% or 82 to 90% or 83 to 88%) by weight starch based on total weight of thickener, with the proviso that the thickener further comprises gum, cellulose or both and where gum makes up from 0.05 to 0.125%, and preferably, from 0.06 to 0.115%, and most preferably, from 0.075 to 0.110% (or 0.8 to 0.105%) by weight of the composition and cellulose makes up from 0.2 to 2.0%, and preferably, from 0.25 to 1.65%, and preferably, from 0.275 to 1.6% (or from 0.28 to 0.8%) by weight of the composition,
wherein the thickener is substantially free of synthetic thickener and the wash composition has a pH of 4.25 or less, and preferably, from 3.4 to 4.2, and most preferably, from 3.65 to 4.15 (or 3.7 to 4.1 or 3.7 to 4 or 3.75 to 3.9) and:
e) the composition has from 3.0 to 16%, and preferably, from 4 to 14%, and most preferably, 4.5 to 12% by weight total surfactant; and
f) the total surfactant comprises from 20 to 99.5%, and preferably, 30 to 95%, and most preferably, 50 to 85% (or 55 to 80% or from 60 to 75%) by weight anionic surfactant based on total weight of anionic, zwitterionic and/or amphoteric surfactant in the composition, the composition is substantially free of soap, paraben, formaldehyde donor, thiol, sulfate and/or isethionate.

2. The wash composition according to claim 1 wherein the anionic surfactant comprises a taurate and the zwitterionic surfactant is present and comprises a betaine.

3. The wash composition according to claim 1 or 2, wherein the composition is a home care composition, hand wash, body wash or face wash.

4. The wash composition according to claim 3 wherein the composition is a hand wash composition.

5. The wash composition according to any one of the preceding claims, wherein the composition further comprises 12-hydroxystearic acid, terpineol, thymol, thiamidol or a mixture thereof.

6. The wash composition according to any one of the preceding claims, wherein the composition is substantially free of synthetic thickener, silicone, hydantoin, paraben, sulfate, and isethionate as a surfactant.

7. The wash composition according to claim 6, wherein the synthetic thickener is ammonium acryloyldimethyltaurate vinylpyrrolidone copolymer, alkyl acrylate crosslinked polymer or a mixture thereof.

8. The wash composition according to any one of the preceding claims, wherein the anionic surfactant is sodium methyl lauroyl taurate and the zwitterionic surfactant is cocamidopropyl betaine.

9. The wash composition according to any one of the preceding claims, wherein the biodegradable thickener has from 8 to 38, and preferably, 9 to 35, and most preferably, 10 to 32 times more starch than cellulose, or gum or the combination of cellulose and gum.

10. The wash composition according to any one of the preceding claims, wherein water makes up from 62 to 95% by weight of the wash composition.

11. The wash composition according to any one of the preceding claims, wherein the wash composition further comprises a water soluble or oil soluble active or both.

12. The wash composition according to any one of the preceding claims, wherein the wash composition comprises 4-ethyl resorcinol, 4-hexyl resorcinol, niacinamide, benzalkonium chloride or a mixture thereof.

13. The wash composition according to any one of the preceding claims, wherein the composition comprises no sulfate, silicone, paraben, isethionate as surfactant, thiol and does comprise sodium benzoate.

14. The wash composition according to any one of the preceding claims, wherein the composition comprises zinc pyrithione, octopirox or a mixture thereof.

15. The wash composition according to any one of the preceding claims, wherein the cellulose is a microcrystalline, carboxymethylcellulose, hydroxymethylcellulose, hydroxypropylcellulose, hydroxypropylmethylcellulose, hydroxyethylcellulose or a mixture thereof and the starch is a hydroxypropyl starch, distarch phosphate or mixture thereof and the gum is carrageenan, xanthan or a mixture thereof.

## Patentansprüche

1. Waschmittelzusammensetzung, umfassend:
a) anionisches Tensid;
b) amphoteres Tensid, zwitterionisches Tensid oder beides;
c) 0 bis 4 Gew.-% nichtionisches Tensid; und
d) 1,5 bis 9,5 Gew.-% Verdickungsmittel, wobei das Verdickungsmittel mehr als 50 Gew.-% Stärke, vorzugsweise mindestens 70 Gew.-% Stärke und höchst bevorzugt mindestens 75 Gew.-% (oder 80 bis 92% oder 82 bis 90% oder 83 bis 88%) Stärke, bezogen auf das Gesamtgewicht an Verdickungsmittel, umfasst, dies mit der Maßgabe, dass das Verdickungsmittel ferner Gummi, Cellulose oder beides umfasst und wobei Gummi 0,05 bis 0,125 Gew.-%, vorzugsweise 0,06 bis 0,115 Gew.-% und höchst bevorzugt 0,075 bis 0,110 Gew.-% (oder 0,8 bis 0,105%) der Zusammensetzung ausmacht und Cellulose 0,2 bis 2,0 Gew.-% und vorzugsweise 0,25 bis 1,65 Gew.-% und bevorzugt 0,275 bis 1,6 Gew.-% (oder 0,28 bis 0,8%) des Gewichts der Zusammensetzung ausmacht,
wobei das Verdickungsmittel im Wesentlichen frei von synthetischem Verdickungsmittel ist und die Waschmittelzusammensetzung einen pH-Wert von 4,25 oder weniger, vorzugsweise von 3,4 bis 4,2 und höchst bevorzugt von 3,65 bis 4,15 (oder 3,7 bis 4,1 oder 3,7 bis 4 oder 3,75 bis 3,9) aufweist; und
e) die Zusammensetzung 3,0 bis 16 Gew.-%, vorzugsweise 4 bis 14 Gew.-% und höchst bevorzugt 4,5 bis 12 Gew.-% gesamtes Tensid aufweist; und
f) das gesamte Tensid umfasst 20 bis 99,5 Gew.-%, vorzugsweise 30 bis 95 Gew.-% und höchst bevorzugt 50 bis 85 Gew.-% (oder 55 bis 80% oder 60 bis 75%) anionisches Tensid umfasst,
bezogen auf das Gesamtgewicht des anionischen, zwitterionischen und/oder amphoteren Tensids in der Zusammensetzung, wobei die Zusammensetzung im Wesentlichen frei von Seife, Paraben, Formaldehydspender, Thiol, Sulfat und/oder Isethionat ist.

2. Waschmittelzusammensetzung nach Anspruch 1, wobei das anionische Tensid ein Taurat umfasst und das zwitterionische Tensid vorhanden ist und ein Betain umfasst.

3. Waschmittelzusammensetzung nach Anspruch 1 oder 2, wobei die Zusammensetzung ein Haushaltsreinigungsmittel, Handwaschmittel, Körperwaschmittel oder Gesichtswaschmittel ist.

4. Waschmittelzusammensetzung nach Anspruch 3, wobei die Zusammensetzung ein Handwaschmittel ist.

5. Waschmittelzusammensetzung nach irgendeinem der vorhergehenden Ansprüche, wobei die Zusammensetzung ferner 12-Hydroxystearinsäure, Terpineol, Thymol, Thiamidol oder eine Mischung davon umfasst.

6. Waschmittelzusammensetzung nach irgendeinem der vorhergehenden Ansprüche, wobei die Zusammensetzung im Wesentlichen frei von synthetischen Verdickungsmitteln, Silikon, Hydantoin, Paraben, Sulfat und Isethionat als ein Tensid ist.

7. Waschmittelzusammensetzung nach Anspruch 6, wobei das synthetische Verdickungsmittel ein Ammoniumacryloyldimethyltaurat-Vinylpyrrolidon-Copolymer, ein Alkylacrylat-vernetztes Polymer oder eine Mischung davon ist.

8. Waschmittelzusammensetzung nach irgendeinem der vorhergehenden Ansprüche, wobei das anionische Tensid Natriummethyl-Lauroyl-Taurat und das zwitterionische Tensid Cocamidopropylbetain ist.

9. Waschmittelzusammensetzung nach irgendeinem der vorhergehenden Ansprüche, wobei das biologisch abbaubare Verdickungsmittel 8 bis 38, vorzugsweise 9 bis 35 und höchst bevorzugt 10 bis 32 Mal mehr Stärke als Cellulose oder Gummi oder die Kombination aus Cellulose und Gummi aufweist.

10. Waschmittelzusammensetzung nach irgendeinem der vorhergehenden Ansprüche, wobei Wasser 62 bis 95 Gew.-% der Waschmittelzusammensetzung ausmacht.

11. Waschmittelzusammensetzung nach irgendeinem der vorhergehenden Ansprüche, wobei die Waschmittelzusammensetzung ferner einen wasserlöslichen oder öllöslichen Wirkstoff oder beides umfasst.

12. Waschmittelzusammensetzung nach irgendeinem der vorhergehenden Ansprüche, wobei die Waschmittelzusammensetzung 4-Ethylresorcin, 4-Hexylresorcin, Niacinamid, Benzalkoniumchlorid oder eine Mischung davon umfasst.

13. Waschmittelzusammensetzung nach irgendeinem der vorhergehenden Ansprüche, wobei die Zusammensetzung kein Sulfat, Silikon, Paraben, Isethionat als Tensid, Thio umfasst und Natriumbenzoat umfasst.

14. Waschmittelzusammensetzung nach irgendeinem der vorhergehenden Ansprüche, wobei die Zusammensetzung Zinkpyrithion, Octopirox oder eine Mischung davon umfasst.

15. Waschmittelzusammensetzung nach irgendeinem der vorhergehenden Ansprüche, wobei die Cellulose eine mikrokristalline Carboxymethylcellulose, Hydroxymethylcellulose, Hydroxypropylcellulose, Hydroxypropylmethylcellulose, Hydroxyethylcellulose oder eine Mischung davon ist und die Stärke eine Hydroxypropylstärke, Distärkephosphat oder eine Mischung davon ist und das Gummi Carrageen, Xanthan oder eine Mischung davon ist.

## Revendications

1. Composition de lavage comprenant :
a) un tensioactif anionique ;
b) un tensioactif amphotère, un tensioactif zwitterionique ou les deux ;
c) 0 à 4 % en poids d'un tensioactif non ionique ; et
d) 1,5 à 9,5 % en poids d'un épaississant, l'épaississant comprenant plus de 50 % en poids d'amidon, et de préférence au moins 70 % en poids d'amidon, et de manière mieux préférée, au moins 75 % en poids (ou 80 à 92 % ou 82 à 90 % ou 83 à 88 %) en poids d'amidon sur la base du poids total de l'épaississant, à condition que l'épaississant comprennent en outre de la gomme, de la cellulose ou les deux, et où la gomme constitue de 0,05 à 0,125 % en poids, et de préférence, de 0,06 à 0,115 %, et de manière mieux préférée, de 0,075 à 0,110 % (ou 0,8 à 0,105 %) en poids de la composition et la cellulose constitue de 0,2 à 2,0 %, et de préférence de 0,25 à 1,65 % et de préférence de 0,275 à 1,6 % (ou de 0,28 à 0,8 %) en poids de la composition,
où l'épaississant est sensiblement exempt d'épaississant synthétique et la composition de lavage a un pH de 4,25 ou moins, et de préférence de 3,4 à 4,2, et de manière mieux préférée, de 3,65 à 4,15 (ou 3,7 à 4,1 ou 3,7 à 4 ou 3,75 à 3,9) et :
e) la composition a de 3,0 à 16 % et de préférence de 4 à 14 % et de manière mieux préférée de 4,5 à 12 % en poids de tensioactif total ; et
f) le tensioactif total comprend de 20 à 99,5 %, et de préférence de 30 à 95 % et de manière mieux préférée de 50 à 85 % (ou 55 à 80 % ou de 60 à 75%) en poids d'un tensioactif anionique sur la base du poids total du tensioactif anionique, zwitterionique et/ou amphotère dans la composition, la composition étant sensiblement exempte de savon, de parabène, de donneur de formaldéhyde, de thiol, de sulfate et/ou d'iséthionate.

2. Composition de lavage selon la revendication 1, dans laquelle le tensioactif anionique comprend un taurate et le tensioactif zwitterionique est présent et comprend une bétaïne.

3. Composition de lavage selon la revendication 1 ou 2, où la composition est une composition de soin ménager, de lavage des mains, de lavage du corps ou de lavage du visage.

4. Composition de lavage selon la revendication 3, où la composition est une composition de lavage des mains.

5. Composition de lavage selon l'une quelconque des revendications précédentes, où la composition comprend en outre de l'acide 12-hydroxystéarique, du terpinéol, du thymol, du thiamidol ou un mélange de ceux-ci.

6. Composition de lavage selon l'une quelconque des revendications précédentes, où la composition est sensiblement exempte d'épaississant synthétique, de silicone, d'hydantoïne, de parabène, de sulfate, et d'iséthionate en tant que tensioactif.

7. Composition de lavage selon la revendication 6, dans laquelle l'épaississant synthétique est un copolymère de vinylpyrrolidone - acryloyldiméthyltaurate d'ammonium, un polymère réticulé par un alkylacrylate ou un mélange de ceux-ci.

8. Composition de lavage selon l'une quelconque des revendications précédentes, dans laquelle le tensioactif anionique est le méthyllauroyltaurate de sodium et le tensioactif zwitterionique est la cocamidopropylbétaïne.

9. Composition de lavage selon l'une quelconque des revendications précédentes, dans laquelle l'épaississant biodégradable a de 8 à 38, et de préférence 9 à 35, et de manière mieux préférée, 10 à 32 fois plus d'amidon que la cellulose, ou la gomme ou la combinaison de cellulose et de gomme.

10. Composition de lavage selon l'une quelconque des revendications précédentes, dans laquelle l'eau constitue de 62 à 95 % en poids de la composition de lavage.

11. Composition de lavage selon l'une quelconque des revendications précédentes, où la composition de lavage comprend en outre un agent actif hydrosoluble ou liposoluble ou les deux.

12. Composition de lavage selon l'une quelconque des revendications précédentes, où la composition de lavage comprend du résorcinol de 4-éthyle, du résorcinol de 4-hexyle, du niacinamide, du chlorure de benzalconium ou un mélange de ceux-ci.

13. Composition de lavage selon l'une quelconque des revendications précédentes, où la composition de lavage ne comprend pas de sulfate, de silicone, de parabène, d'iséthionate en tant que tensioactif, de thiol mais comprend du benzoate de sodium.

14. Composition de lavage selon l'une quelconque des revendications précédentes, où la composition comprend de la pyrithione de zinc, de l'octopirox ou un mélange de ceux-ci.

15. Composition de lavage selon l'une quelconque des revendications précédentes, dans laquelle la cellulose est une cellulose microcristalline, carboxyméthylcellulose, hydroxyméthylcellulose, hydroxypropylcellulose, hydroxypropylméthylcellulose, hydroxyéthylcellulose ou un mélange de celles-ci et l'amidon est un amidon d'hydroxypropyle, un diphosphate d'amidon ou un mélange de ceux-ci et la gomme est la carraghénane, la xanthane ou un mélange de celles-ci.
